# EUROPÄISCHE PATENTSCHRIFT

(11) **EP 1 978 896 B1**
(45) Veröffentlichungstag und Bekanntmachung des Hinweises auf die Patenterteilung: **22.07.2009**
(21) Anmeldenummer: 07712061.6
(22) Anmeldetag: 19.01.2007
(51) Int. Cl.: A61F 2/30, A61F 2/40

(54) **HUMERUSKOMPONENTE**
HUMERAL COMPONENT
COMPOSANT D'HUMÉRUS

(30) Priorität: 20.01.2006 EP 06100680; 27.04.2006 CH 698062006
(43) Veröffentlichungstag der Anmeldung: 15.10.2008
(73) Patentinhaber: Zimmer GmbH, 8404 Winterthur (CH)
(72) Erfinder: FORRER, Michael, CH-8472 Seuzach (CH)
(86) Internationale Anmeldenummer: PCT/EP2007/050524
(87) Internationale Veröffentlichungsnummer: WO 2007/082925

(56) Entgegenhaltungen:
- EP-A- 1 093 777
- EP-A- 1 216 668
- EP-A- 1 415 621
- EP-A- 1 527 757
- DE-U1- 29 918 589
- GB-A- 2 268 408
- US-A- 4 608 052
- US-A- 4 963 155
- US-A1- 2004 054 420
- US-A1- 2004 181 286

## Beschreibung

Die vorliegende Erfindung betrifft eine Humeruskomponente einer Schultergelenkprothese.

Die Verwendung von Schultergelenkprothesen kann erforderlich sein, wenn das Schultergelenk eines Patienten geschädigt ist und daher Schmerzen verursacht und/oder das Gelenk in seiner Funktionsfähigkeit eingeschränkt ist.

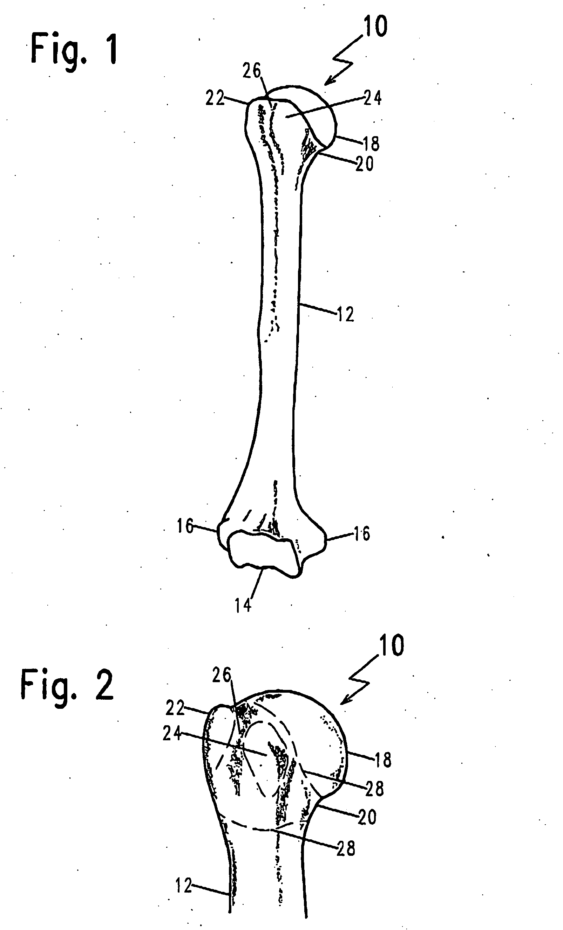

In Fig. 1 ist ein rechter Humerus 10 (Oberarmknochen) schematisch in einer Ansicht von vorne dargestellt. Er gliedert sich in den Humerusschaft 12 und ein proximales Ende und ein distales Ende. Das distale Ende des Humerus 10 wird im Wesentlichen von der Humeruskondyle 14 (Condylus humeri) und den medial und lateral angeordneten Epikondylen 16 gebildet. Die walzenförmige Humeruskondyle 14 dient der gelenkigen Verbindung mit dem Unterarmknochen (nicht gezeigt). Das proximale Ende des Humerus 10 setzt sich im Wesentlichen aus einem Humeruskopf 18 (Caput humeri), einem Humerushals 20 (Collum anatomicum) und einem großen Rollhügel 22 (Tuberculum majus) sowie einem kleinen Rollhügel 24 (Tuberculum minus) zusammen. Zwischen den beiden Rollhügeln 22, 24 liegt eine Rollhügelrinne 26 (Sulcus intertubercularis).

Die knöchernen Strukturen des Schultergelenks (Glanohumeral-Gelenk) besteht aus dem annähernd kugelförmigen Humeruskopf 18 und der Gelenkpfanne des Schulterblatts (Glenoid, nicht gezeigt). Die Gelenkpfanne ist vergleichsweise flach, so dass die Kontaktfläche zu dem gegenüberliegenden Humeruskopf 18 relativ klein ist. Da keine stark ausgeprägten Bänder vorhanden sind, müssen die Muskeln, die das Gelenk umhüllen, dieses stabilisieren. Man spricht in diesem Fall von einem muskelgesicherten Gelenk.

Bei degenerativen Gelenkerkrankungen, beispielsweise durch Verschleiß (Arthrose), oder entzündlichen Gelenkerkrankungen (Arthritis) kann in vielen Fällen ein Oberflächenersatz für den Humeruskopf 18 angezeigt sein. Dieser ist auch bei Humeruskopfnekrose indiziert. Bei fortschreitender Schädigung des proximalen Humerus kann es unter Umständen notwendig sein, auf eine primäre Totalendoprothese zurückzugreifen.

In Folge von Unfällen treten auch häufig Frakturen im Bereich des proximalen Humerus 18 auf, die nicht durch funktionserhaltende Operationen behandelt werden können. Je nach Schwere der Verletzung sind oftmals auch die versorgenden Gefäße des Humeruskopf 18 zerstört, so dass mit dem Absterben des Humeruskopf 18 gerechnet werden muss. In diesen Fällen besteht die Möglichkeit, den Humeruskopf 18 durch eine Prothese zu ersetzen.

Frakturen im Bereich des proximalen Humerus werden in Abhängigkeit von der Anzahl der entstandenen Fragmente, des Ausmaßes der Fragmentverschiebung und des Frakturverlaufs in unterschiedliche Typen eingeteilt. "Einteil-Frakturen", d.h. Traumata mit im Wesentlichen lediglich einer Bruchfläche, ohne Verschiebung der einzelnen Fragmente gegeneinander werden als Typ-0-Frakturen bezeichnet und müssen meist nicht operativ behandelt werden. Typ-A-Frakturen weisen zwei Fragmente auf und werden häufig durch den Abriss des großen Rollhügels 22 oder des kleinen Rollhügels 24 charakterisiert. Frakturen des Typs B, C und X unterscheiden sich in der Lage der Bruchflächen und weisen zumeist zwei bis vier Knochenfragmente auf. Bei diesen Frakturtypen, sowie der Humeruskopf-Trümmerfraktur, muss der proximale Bereich des Humerus mittels Drähten, Schrauben oder Platten stabilisiert werden. In schweren Fällen kann auch, wie vorstehend kurz erwähnt, die Verwendung einer Schultergelenkprothese angezeigt sein.

Fig. 2 verdeutlicht anhand der gestrichelten Linien typische Bruchkanten 28, wie sie bei Frakturen im Bereich des proximalen Humerus 10 auftreten. Besonders häufig treten Abrisse der Rollhügel 22, 24 und/oder Frakturen in Bereich des Humerushalses 20 und des proximalen Humerusschafts 12 auf.

Grundsätzlich sind Prothesen zur Wiederherstellung der Funktionalität des Schultergelenks bekannt.

Die EP 1415621 beschreibt eine Schultergelenkprothese mit zwei zusammenwirkenden Lagerkörpern, einem Schaft und einer Kupplung zur Verbindung des Schafts mit einem humerusseitigen Lagerkörper. Dieses System ist sehr flexibel und erlaubt die Wahl eines für den jeweiligen Fall geeigneten Lagerkörpers. So kann der Lagerkörper beispielsweise die Anatomie des Humeruskopfs nachbilden. Gemäß einer alternativen Ausführungsform kann aber auch die Funktion von Kugel- und Lagerschale vertauscht sein. Außerdem lassen sich bei der bekannten Humeruskopf-Prothese unterschiedliche Stellungen des Lagerkörpers relativ zum Schaft realisieren, um den individuellen anatomischen Anforderungen der unterschiedlichen Patienten Rechnung zu tragen.

Darüber hinaus ist aus der EP 1093777 eine Schulterendoprothese bekannt, die verschiedene Elemente zur Fixierung von Knochenfragmenten aufweist. Diese Elemente können beispielsweise Laschen, Ösen, Krallen und Bohrungen sein.

Aus EP 1 216 668 ist eine Prothese mit einem Schaft bekannt, der eine Oberflächenstruktur in Form von langgezogenen Rippen aufweist, die sich im Wesentlichen in Umfangsrichtung um eine in proximal-distaler Richtung verlaufende Achse herum erstrecken.

Aus EP 1 415 621 ist eine Schultergekentes - prothese bekountgeworden, welche unterhalb des lagerkörpers einen Stützkgser aufweist, welcher das Zusammenwochsen von knachen fragmenten begünstigen soll.

US 4,608,052 oftenbart eind Prothese mit einem sclaft, welche in einem zwischen einemschaft und einem lagerkörfer gelegenen Bereich eine strakfur aufweist, welde das Ein wodsin von Gewebe begünstigen soll.

Es soll nunmehr eine neuartige Humeruskomponente einer Schultergelenkprothese angegeben werden. Die nachfolgend beschriebene Humeruskomponente ermöglicht, bei weitgehendem Erhalt von Knochensubstanz, die Rekonstruktion des Schultergelenks und damit eine Wiederherstellung der normalen Kinematik. Neben einer Reihe weiterer Eigenschaften lässt sich die Humeruskomponente sicher und zuverlässig im Humerusschaft fixieren und erlaubt darüber hinaus die zuverlässige Fixierung von Knochenfragmenten des proximalen Humerus an der Humeruskomponente. Somit kann eine gute Verankerung des den Humeruskopf ersetzenden Lagerkörpers gewährleistet werden.

Die hier angegebene Humeruskomponente einer Schultergelenkprothese umfasst einen Lagerkörper, einen Schaft und einen Übergangsbereich. Der Übergangsbereich ist zwischen dem Lagerkörper und dem Schaft angeordnet. Der Schaft der Humeruskomponente ist dazu vorgesehen, um in einen Humerus implantiert zu werden. Der Übergangsbereich der Humeruskomponente, das heißt der Bereich, der im Wesentlichen die Metaphyse und Teile der Epiphyse des Humerus umfasst, ist großflächig mit einer Struktur in Form von Erhebungen versehen, wobei die Erhebungen auf der Oberfläche des Übergangsbereiches sowohl im Wesentlichen in proximal-distaler Richtung als auch im Wesentlichen in Umfangsrichtung diskret verteilt angeordnet sind.

Die Erhebungen bilden also keine langgezogenen rippenartigen Oberflächenstrukturen, sondern eine prinzipiell beliebige diskrete Verteilung auf der Oberfläche, wobei eine strenge Ausrichtung der Erhebungen in proximal-distaler Richtung und - in einem Querschnitt senkrecht zur proximal-distalen Richtung gesehen - in Umfangsrichtung nicht zwingend ist. Vielmehr ist unter "proximal-distaler Richtung" sowie "Umfangsrichtung" auch eine zufällige Verteilung der Erhebungen auf der Oberfläche, also gewissermaßen eine diskrete Anordnung der Erhebungen in allen Richtungen, zu verstehen. Anders ausgedrückt kann vorliegend auch von punktuellen oder "punktförmigen", insbesondere im Wesentlichen punktförmig zulaufenden, Spitzen gesprochen werden, im Gegensatz zu rippenartigen und damit linienförmigen Erhebungen.

Diese Struktur erleichtert die Fixierung von Knochenfragmenten, wie sie typischerweise bei Humerusfrakturen auftreten, insbesondere wenn mehrere Fragmente vorliegen.

Außerdem wird durch die Struktur der Ansatz von Muskeln und Sehnen an der Prothese unterstützt. Dies ist gerade bei Prothesen des Schultergelenks von besonderer Bedeutung, da, wie vorstehend bereits ausgeführt, die Stabilität des Schultergelenks wesentlich von den das Gelenk umhüllenden Muskeln abhängt.

Zusätzlich ist vorgesehen, dass zwischen dem Übergangsbereich und dem Lagerkörper, der beispielsweise als Lagerkugel oder Lagerschale ausgestaltet sein kann, eine metaphysäre Basisplatte angeordnet ist. Diese Basisplatte weist ebenfalls Erhebungen zum Ansatz von Knochenfragmenten und/oder Muskelansätzen und/oder Sehnenansätzen auf. Die Basisplatte kann zusätzliche Stützfunktionen erfüllen und durch ihren Unterbau das Zusammenwachsen der Knochenfragmente und Muskel- bzw. Sehnenansätze begünstigen.

Somit ist also eine hohe Primär- und Langzeitstabilität der Humeruskomponente gewährleistet.

Weitere Ausführungsformen der Erfindung sind in den abhängigen Ansprüchen, der Beschreibung und den Zeichnungen angegeben.

Die Humeruskomponente kann sowohl an ihrer anterioren und ihrer posterioren Oberfläche des Übergangsbereichs im Wesentlichen vollständig eine Struktur in Form von Erhebungen aufweisen. Außerdem kann die laterale Oberfläche des Übergangsbereichs ebenso mit Erhebungen versehen sein. Eine derartige Humeruskomponente trägt den typischen Frakturgeometrien Rechnung und ermöglicht die sichere Fixierung sowohl des großen, als auch des kleinen Rollhügels und anderer Knochenfragmente.

In einer Ausführungsform erstreckt sich der Übergangsbereich über zumindest 10 %, insbesondere wenigstens 15 % und weiter insbesondere wenigstens 20 %, der proximal-distalen Gesamtlänge der Humeruskomponente. Durch Vorsehen dieser proximal-distalen Erstreckung des Übergangsbereichs wird ein sehr guter Halt der zu befestigenden Knochenfragmente und/ oder Muskel-/Sehnenansätze sichergestellt.

Insbesondere ist der Übergangsbereich vollständig über seine gesamte proximal-distale Erstreckung mit den Erhebungen versehen.

Es kann vorgesehen sein, dass die Struktur auf einem Umfangsbereich eines Querschnitts senkrecht zur proximal-distalen Erstreckung der Humeruskomponente von wenigstens 240° vorgesehen ist. Insbesondere kann dieser Umfangsbereich auch wenigstens 270° umfassen. Mit anderen Worten können die die Struktur ausbildenden Strukturelemente in einem streifenförmigen oder streifenartigen Bereich angeordnet sein, der sich über einen großen Teil des Umfangs erstreckt. Durch die Anordnung von Erhebungen in einem derart großen Umfangsbereich wird die Primärstabilität des rekonstruierten proximalen Humerusendes verbessert. Gerade wenn eine Mehrzahl von Knochenfragmenten vorliegt, die von verschiedenen Seiten an der Prothese zu fixieren sind, ist eine derartige Ausgestaltung des Übergangsbereichs der Humeruskomponente von Vorteil.

In einer Ausführungsform umfasst der Übergangsbereich den metaphysären Bereich der Humeruskomponente, die bei dem gesunden Gelenk bzw. bei der vollständigen Gelenkprothese zwischen Epiphyse und Diaphyse angeordnet ist.

Gemäß einer Ausführungsform der Humeruskomponente sind die Erhebungen im Wesentlichen regelmäßig geformt. Diese Erhebungen können beispielsweise im Wesentlichen die Form von Pyramiden mit rechteckiger bzw. quadratischer Basis und/oder die Form von Kegeln mit kreisförmiger, ovaler bzw. elliptischer Basis aufweisen. Weiterhin können die Erhebungen im Wesentlichen regelmäßig auf der Oberfläche des Übergangsbereichs verteilt angeordnet sein.

Mit diesen Maßnahmen, die einzeln oder in Kombination umgesetzt werden können, wird die Oberfläche hinsichtlich ihrer funktionellen Eigenschaften optimiert, das heißt der Ansatz von Muskeln und Sehnen wird erleichtert und die Fixierung von Knochenfragmenten komplizierter traumatischer Schädigungen des proximalen Humerus lässt sich einfach und sicher durchführen. Außerdem gewährleisten sie eine kostengünstige Fertigung der Prothese, die trotzdem hohen Qualitätsanforderungen genügt.

Es kann weiterhin vorgesehen sein, dass der Lagerkörper und der Übergangsbereich trennbar miteinander verbunden sind. Durch diesen modularen Aufbau können die für den spezifischen Fall optimalen Komponenten kombiniert werden. So steht eine große Auswahl an Schaft-, symmetrischer und asymmetrischer Kopf- und Glenoidkomponenten zur Verfügung und ermöglicht eine ideale Wiederherstellung der Anatomie des Schultergelenks. Außerdem ist ein modulares System leichter zu implantieren und anzupassen.

In einer weiteren Ausführungsform weist die metaphysäre Basisplatte eine äußere Stützfläche auf, die eine zwischen dem Lagerkörper und dem Schaft vorhandene Lücke zumindest teilweise ausfüllt. Diese äußere Stützfläche kann zumindest teilweise ebenfalls eine Struktur in Form von Erhebungen aufweisen. Somit ergibt sich durch die Stützfläche zusammen mit der Fläche des Übergangsbereichs eine im Wesentlichen geschlossene Fläche, die sich an die Unterseite des Lagerkörpers anschließt. Eine derartige geschlossene Ausführungsform unterstützt das Zusammenwachsen der natürlichen Komponenten der betroffenen Körperregion. Durch die Erhebungen auf der Stützfläche wird die Verankerung der Humeruskomponente zusätzlich verbessert.

Die äußere Stützfläche der metaphysären Basisplatte kann eine laterale Nut aufweisen. Eine laterale Nut kann auch in einem proximalen Bereich des Übergangsbereichs angeordnet sein. Derartige laterale Nuten sind für die Befestigung von Tuberositas (Knochenvorsprünge mit rauer Oberfläche, an welchen die Sehnen von Muskeln ansetzen) vorgesehen. Eine oder mehrere Bohrungen, die benachbart und/oder unmittelbar an der Nut angeordnet sein können, erleichtern die Befestigung der Tuberositas, damit diese zuverlässig und anatomisch korrekt unterhalb des Humeruskopf bzw. seiner Prothese angeordnet werden können. Dadurch werden "Impingements" und Dislokationen der Rollhügel vermieden. Als "Impingement" ("Engpaß") bezeichnet man in diesem Zusammenhang das Einklemmen von Sehnen, knorpeligen Gelenklippen oder Schleimhautfalten im Bereich von Gelenken, das zu einer schmerzhaften Funktionsstörung führt. Impingement-Syndrome betreffen häufig das Schultergelenk als Engpaßsyndrom der Rotatorenmanschette, d.h. der Muskelgruppe, die den Kopf des Humerus in der sehr flachen Gelenkpfanne des Schulterblatts hält.

Gemäß einer Ausführungsform sind der Lagerköper und die metaphysäre Basisplatte und/oder die metaphysäre Basisplatte und der Übergangsbereich trennbar miteinander verbunden. Die grundsätzlichen Vorteile der modularen Bauweise wurden bereits vorstehend ausgeführt. Darüber hinaus erleichtert die modulare Basisplatte den Austausch des Lagerkörpers. Damit lassen sich Revisions- und Anpassungsoperationen einfacher und schonender ausführen.

In einer weiteren Ausführungsform ist der Übergangsbereich frei von vor-/abstehenden Fixierungslaschen oder -ösen ausgebildet. Derartige Laschen oder Ösen sind teuer zu fertigen und außerdem schwierig zu handhaben.

Gemäß einer Ausführungsform weist die Humeruskomponente im Übergangsbereich Bohrungen zur Fixierung von Knochenfragmenten mit Hilfe von Zugfäden oder -drähten auf. Im Vergleich zu von der Oberfläche des Übergangsbereichs abstehenden Laschen oder Ösen können durch die Verwendung der Bohrungen Knochenfragmente mit Hilfe von Drähten oder Fäden besser an den Übergangsbereich gedrückt werden. Durch dieses Anpressen entfaltet sich auf vorteilhafte Weise die fixierende Wirkung der Erhebungen. Mit anderen Worten wird somit eine größere Anpresskraft der Knochenfragmente erreicht.

Die Erfindung betrifft weiterhin eine Schultergelenkprothese, welche eine Humeruskomponente gemäß zumindest einer der oben beschriebenen Ausführungsformen umfasst. Eine derartige Schulterprothese zeichnet sich durch eine hohe Primärstabilität aus und erlaubt eine gute Rekonstruktion der Funktionalität des Glenohumeral-Gelenks. Das Prothesendesign, insbesondere die Ausgestaltung des Übergangsbereichs der Humeruskomponente, unterstützt das Zusammenwachsen von Knochenfragmenten im proximalen Bereich des Humerus.

Alle sowohl in den Ansprüchen als auch in der Beschreibung und in den Zeichnungen in Verbindung mit der Humeruskomponente ggf. verwendeten und gemäß der fachüblichen Konvention gewählten Ausrichtungs-, Positionierungs-, Orientierungs- und Richtungsangaben, die insbesondere anatomische Achsen, Ebenen, Richtungen im Raum und Bewegungsrichtungen betreffen, sind dem Fachmann bekannt und beziehen sich auf den implantierten Zustand der Humeruskomponente.

Die Erfindung wird im Folgenden rein beispielhaft anhand möglicher Ausführungsformen und unter Bezugnahme auf die Zeichnungen beschrieben.
- Fig. 1: zeigt eine schematische Darstellung eines rechten Humerus in einer Ansicht von anterior (siehe Einleitung).
- Fig. 2: zeigt eine schematische Darstellung des proximalen Endes eines rechten Humerus mit typischen Bruchkanten bei einer traumatischen Schädigung in einer Ansicht von anterior (siehe Einleitung).
- Fig. 3a: zeigt eine schematische Darstellung einer Ausführungsform einer proximalen rechten Humeruskomponente in einer perspektivischen Ansicht von anterior.
- Fig. 3b: zeigt die in Fig. 3a dargestellte Ausführungsform einer proximalen rechten Humeruskomponente in einer perspek-tivischen Ansicht schräg von anterior.
- Fig. 3c: zeigt die in Fig. 3a dargestellte Ausführungsform einer proximalen Humeruskomponente in einer perspektivischen Ansicht von lateral.
- Fig. 3d: zeigt die in Fig. 3a dargestellte Ausführungsform einer proximalen Humeruskomponente in einer Explosionszeichnung.
- Fig. 4: zeigt eine vergrößerte Darstellung von Fig. 3d.
- Fig. 5: zeigt eine vergrößerte Darstellung von Fig. 3a.
- Fig. 6: zeigt eine vergrößerte Darstellung von Fig. 3b.
- Fig. 7: zeigt eine vergrößerte Darstellung von Fig. 3c.
- Fig. 8: zeigt eine Ausführungsform einer proximalen rechten Humeruskomponente in einer perspektivischen Ansicht schräg von posterior in einer Explosionszeichnung.
- Fig. 9: zeigt eine mögliche Ausführungsform einer Erhebung der Struktur.

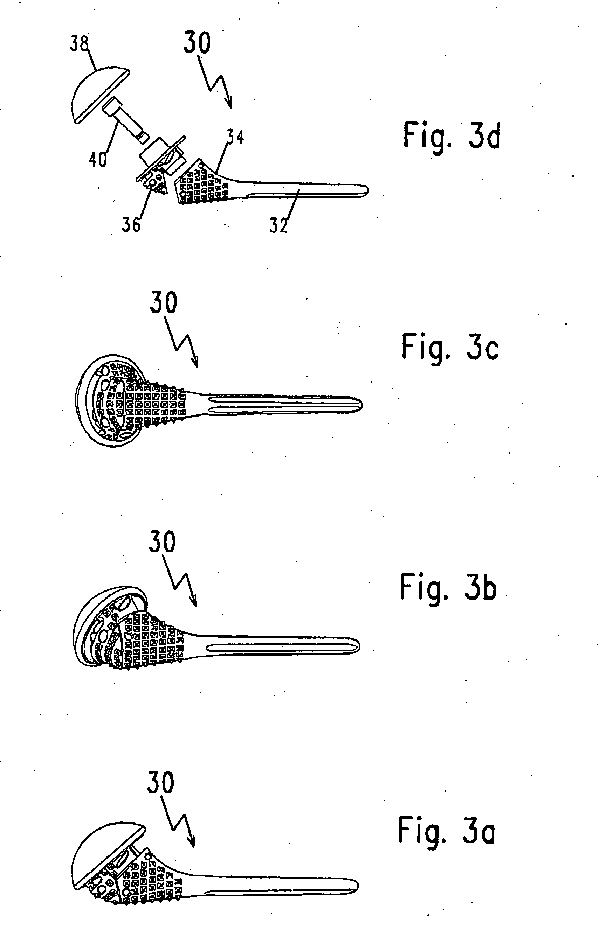

Die Fig. 3a bis 3c sind verschiedene Ansichten von Ausführungsformen einer Humeruskomponente 30. Wie aus Fig. 3d deutlich ersichtlich, gliedert sich die Humeruskomponente 30 im Wesentlichen in einen Schaft 32, einen Übergangsbereich 34, eine metaphysäre Basisplatte 36 und einen Lagerkopf 38, wobei der Schaft 32 und der Übergangsbereich 34 eine Einheit bilden. Diese drei einzelnen Baueinheiten der Humeruskomponente 30 können zusammengefügt und durch eine Spannschraube 40 fixiert werden. Eine detaillierte Beschreibung der dargestellten Ausführungsform wird anhand der folgenden Figuren gegeben.

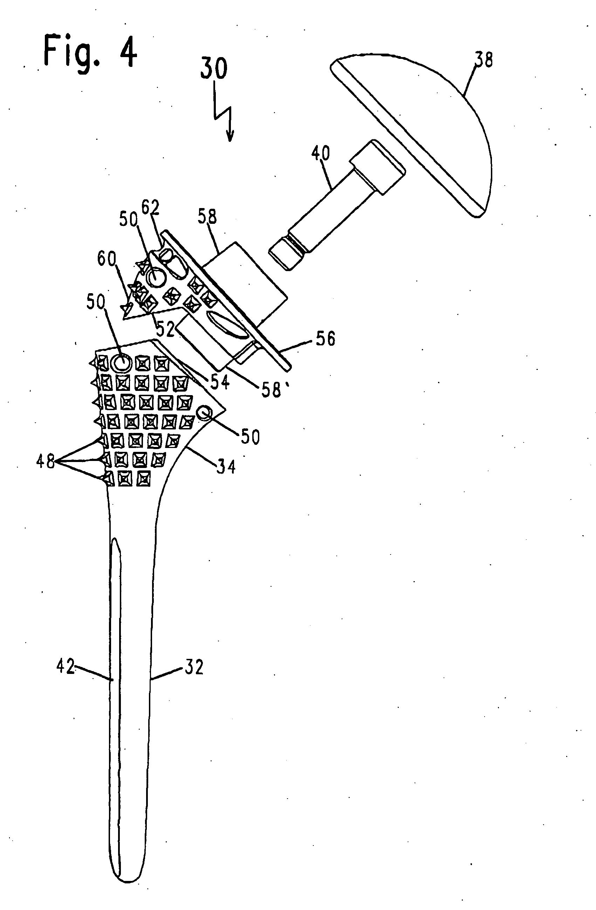

Fig. 4 ist eine vergrößerte Ansicht von Fig. 3d. Der Humerusschaft 32 ist zur Implantation in einen Humerus 10 vorgesehen. Es ist eine Längsnut 42 zu sehen, die unter anderem zur rotationsfesten Fixierung des Schafts 32 in einem Humerus 10 dient. Der Schaft 32 weist eine leicht konische Geometrie auf (1.5°) und nimmt im Verlauf von distal nach proximal an Umfang zu. Proximal schließt sich an den Schaft 32 der Übergangsbereich 34 an. Der Übergangsbereich 34 weist eine strukturierte Oberfläche mit pyramidenförmigen Erhebungen 48 auf. Die Erhebungen 48 tragen zur Verbesserung der Primärstabilität der Prothese bei, indem sie die Knochenfragmente fixieren und den Ansatz der Muskeln fördern. Zusätzlich ist die Oberfläche grob gestrahlt.

In der hier gezeigten Ausführungsform sind die pyramidenförmigen Erhebungen 48 gleichmäßig verteilt. In anderen Ausführungsformen kann die Verteilung der Erhebungen 48 auch ungleichmäßig sein. Beispielsweise können Bereiche, in denen eine erhöhte Belastung der Knochenfragmente zu erwarten ist, eine erhöhte Erhebungsdichte aufweisen. Auch können die Erhebungen 48 im Gegensatz zu der hier dargestellten Ausführungsform je nach ihrer Position unterschiedliche Formen, Geometrien und Erstreckungen aufweisen. Kegelförmige oder anders geartete Erhebungen, wie auch Kegel- oder Pyramidenstümpfe, sind ebenfalls denkbar.

Der Übergangsbereich 34 ist überdies mit Bohrungen 50 zur Fixierung der Knochenfragmente, beispielsweise abgerissener Rollhügel, an dem Schaft versehen. Dies kann beispielsweise mit Hilfe von Drähten oder Fäden erfolgen.

Die Erhebungen 48 sind auf der anterioren, der posterioren und der lateralen Oberfläche des Übergangsbereichs 34 angeordnet. Diese Ausführungsform weist medial keine Erhebungen auf. Eine derartige Verteilung hat sich für die typischerweise auftretenden Frakturgeometrien als geeignet herausgestellt und ermöglicht eine sehr gute Fixierung der Knochenfragmente. Für besonders gelagerte Fälle kann auch der mediale Abschnitt des Übergangsbereichs 34 mit Erhebungen 48 versehen sein.

Mit anderen Worten erstreckt sich die Struktur in Form von Erhebungen 48 über einen Großteil des Umfangsbereichs des Übergangsbereichs 34 der Humeruskomponente 30. Es kann vorgesehen sein, nicht den gesamten Übergangsbereich 34 in seiner distal-proximalen Längserstreckung mit Erhebungen 48 zu versehen, sondern beispielsweise einen streifenförmigen Bereich mit Erhebungen 48 vorzusehen, der sich über einen großen Teil des Umfangs des Übergangsbereichs 34 erstreckt. Dadurch können Knochenfragmente von praktisch allen Seiten (lateral, medial, anterior und posterior) angeheftet werden.

In der dargestellten Ausführungsform haben die Erhebungen die Form von Pyramiden mit einer quadratischen Basis. Die Höhe der Erhebungen beträgt weniger als 2.5 mm, kann grundsätzlich aber auch weniger als 2 mm betragen. Es können Ausführungsformen mit Erhebungshöhen von mehr als 0.5 mm und insbesondere mehr als 1 mm und beispielsweise 1.5 mm vorgesehen sein. Der Flankenwinkel der Pyramiden beträgt bei der dargestellten Ausführungsform etwa 60°.

Der Übergangsbereich 34 weist an seinem proximalen Ende überdies geeignete Vertiefungen auf, um eine Verbindung mit der metaphysären Basisplatte 36 oder direkt mit einem Lagerkopf 38 herstellen zu können. Außerdem ist ein Gewinde für die Spannschraube 40 vorgesehen. Die Vertiefungen und das Gewinde sind aus dieser Abbildung nicht ersichtlich.

Der Übergangsbereich 34 kann proximal mit der metaphysären Basisplatte 36 verbunden werden. Die metaphysäre Basisplatte 36 weist an ihrem distalen Ende eine Montagefläche 52 auf, welche zu der Montagefläche 54 am proximalen Ende des Übergangsbereichs 34 komplementär ist. An ihrem proximalen Ende weist die metaphysäre Basisplatte 36 einen kreis- oder ellipsenförmigen Plattenabschnitt 56 auf. Die metaphysäre Basisplatte weist weiterhin zwei Zapfen 58, 58' auf, die in komplementäre Ausnehmungen in dem Übergangsbereich 46 und dem Lagerkopf 38 einführbar sind. Der zu dem Übergangsbereich 34 hin weisende distale Zapfen 58' kann beispielsweise einen polygonalen Querschnitt aufweisen.

Darüber hinaus ist die metaphysäre Basisplatte 36 mit einer Stützfläche 60 versehen, welche an ihrer Oberfläche ebenfalls Erhebungen 48 aufweist. Die Stützfläche 60 ist mit einer Bohrung 50 und einer lateralen Nut 62 versehen. Die laterale Nut 62 ermöglicht zusätzliche Verstemmungen und/oder das Anbringen von Knochenfragmenten, wie beispielsweise von Tuberositas. Zur verbesserten Fixierung von den in diesem Bereich angebrachten Knochenstücken ist die Bohrung 50 in unmittelbarer Nähe zu der lateralen Nut 62 angeordnet. Die Fixierung kann mittels durch die Bohrung 50 geführter Drähte/Fäden erfolgen. Es können auch mehrere Bohrungen benachbart zu der lateralen Nut 62 vorgesehen sein. In dieser Ausführungsform hat die Oberfläche der Stützfläche 60 sowohl eine laterale als auch eine anteriore und eine posteriore Komponente.

Die Einzelteile der Humeruskomponente 30 werden mittels der Spannschraube 40, welche in einer Ausführungsform ein metrisches Gewinde aufweist, miteinander verspannt.

Die vorstehend beschriebene Humeruskomponente 30 ist ausgelegt für anatomische Rekonstruktionen von 4-Fragmentfrakturen, wobei besonders der proximale Teil derart optimiert ist, um möglichst genau die natürliche Anatomie nachzubilden. Je nach Schwere der Verletzung und in Abhängigkeit von den anatomischen Gegebenheiten des Patienten und der Frakturgeometrie kann die Humeruskomponente 30 unterschiedlich ausgestaltet sein, wobei auch die Lage und Form der Erhebungen sowie der Bohrungen variieren kann. Unterschiedliche Versionen der Humeruskomponente 30 für die linke und die rechte Schulter sind vorgesehen.

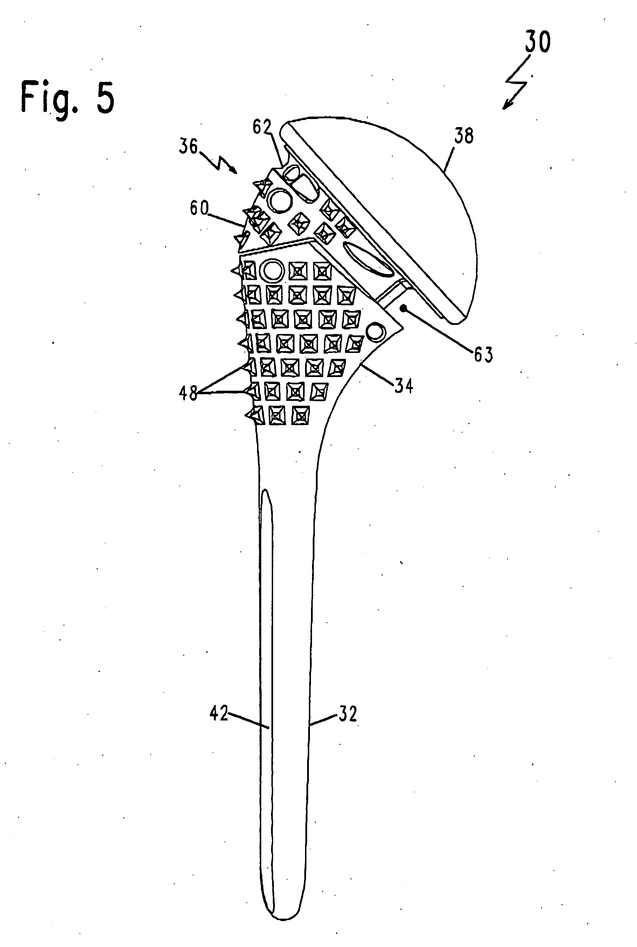

Fig. 5 zeigt eine Ausführungsform der Humeruskomponente 30 in einer Ansicht von anterior in einem zusammengesetzten Zustand. Es ist eine mediale Aussparung 63 an der metaphysären Basisplatte 36 zu erkennen. Sie ermöglicht es, eine eventuell notwendige Resektion noch vorhandenen Knochens zu minimieren und gleichzeitig die Verankerung der Prothese im Knochen zu verbessern.

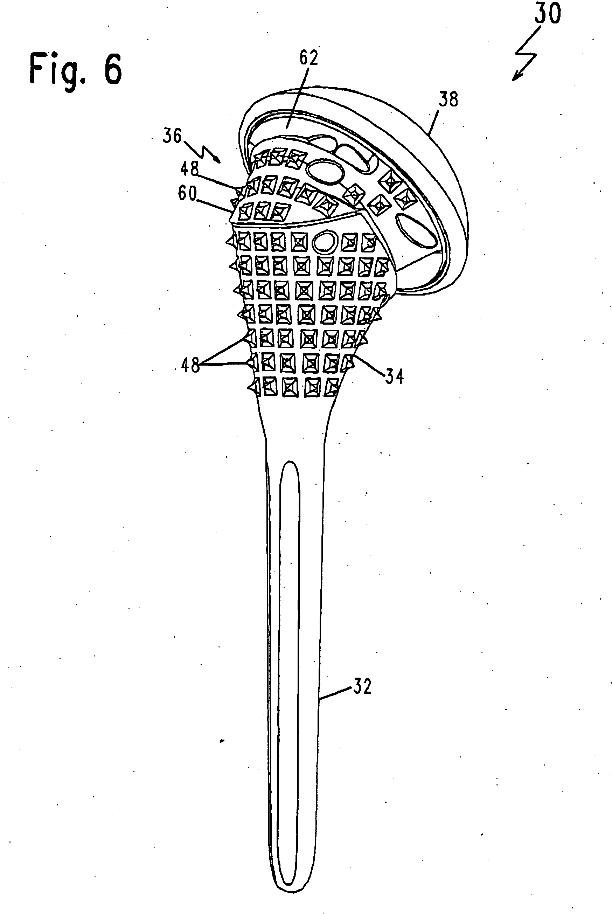

Fig. 6 zeigt die Humeruskomponente 30 in einer Ansicht von schräg vorne und verdeutlicht die großflächige Anordnung von Erhebungen 48 sowohl auf dem Übergangsbereich 34 als auch auf der Stützfläche 60 der metaphysären Basisplatte 36. Außerdem ist aus dieser Figur ersichtlich, dass die distalen Kanten des Lagerkopfs 38 abgerundet sind, um die das Gelenk umhüllenden Muskeln nicht zu schädigen.

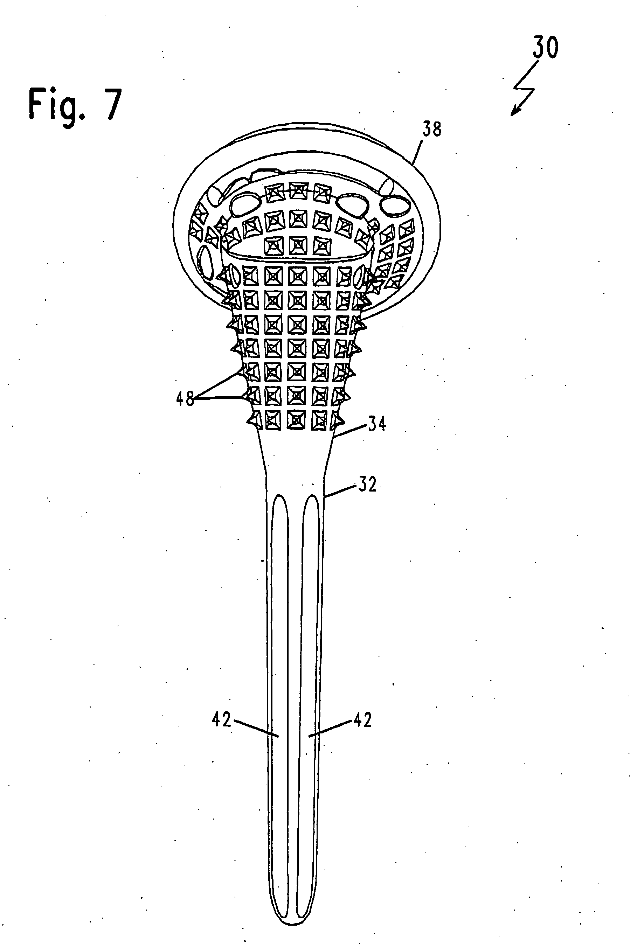

Eine laterale Ansicht der Humeruskomponente 30 geht aus Fig. 7 hervor. Zu sehen ist, dass der Schaft 32 in seinem lateralen Bereich zwei Nuten 42 zur Sicherung der Rotationsstabilität der Humeruskomponente aufweist.

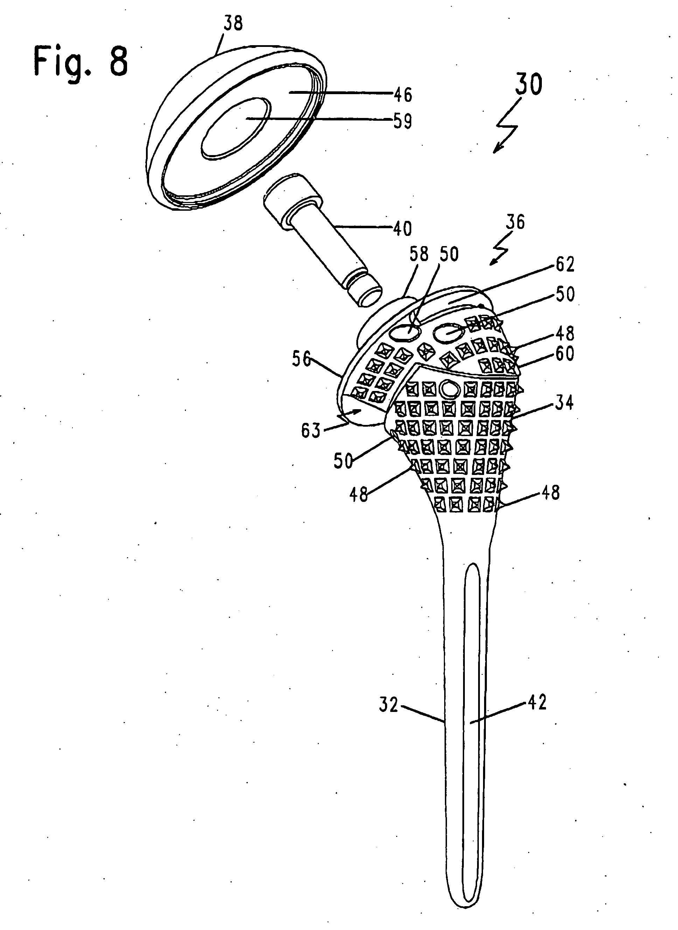

Fig. 8 zeigt eine weitere Darstellung der Humeruskomponente 30. In dieser Ansicht ist eine zurückgesetzte Lagermontagefläche 46 zu sehen. Die Lagermontagefläche 46 bildet eine Ausnehmung, in welche der Plattenabschnitt 56 der metaphysären Basisplatte 36 bei der Montage versenkt wird. Außerdem weist der Lagerkopf 38 eine Montagebohrung 59 zur Aufnahme des Zapfens 58 auf.

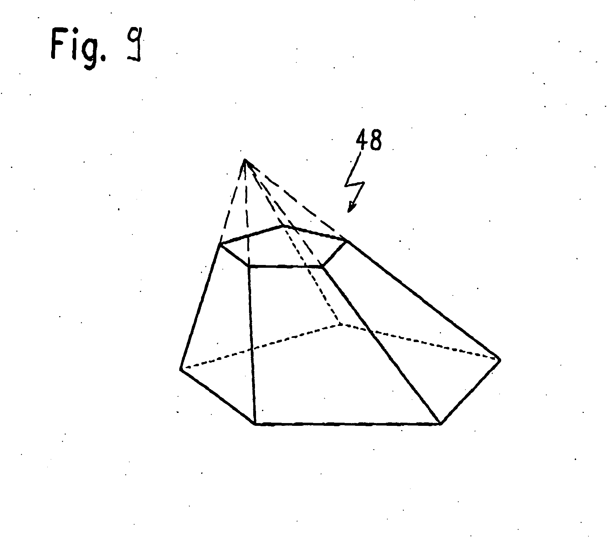

Fig. 9 zeigt eine mögliche Ausführungsform einer Erhebung 48. Dargestellt ist ein Pyramidenstumpf mit unregelmäßiger Basis, der Flanken mit unterschiedlichen Flankenwinkeln aufweist. Grundsätzlich können auch unregelmäßige Kegel und/oder Kegelstümpfe vorgesehen sein. Erhebungen mit unterschiedlichen Flankensteigungen können beispielsweise an Positionen angeordnet werden, an denen hauptsächlich eine gerichtete Belastung zu erwarten ist.

Je nach Anforderungen kann die Formgebung und das Material der Humeruskomponente 30 für zementierte und unzementierte Anwendungen optimiert werden.

### Bezugszeichenliste

- 10: Humerus
- 12: Humerusschaft
- 14: Humeruskondyle
- 16: Epikondyle
- 18: Humeruskopf
- 20: Humerushals
- 22: großer Rollhügel
- 24: kleiner Rollhügel
- 26: Rollhügelrinne
- 28: Bruchkanten
- 30: Humeruskomponente
- 32: Schaft
- 34: Übergangsbereich
- 36: metaphysäre Basisplatte
- 38: Lagerkopf
- 40: Spannschraube
- 42: Längsnut
- 46: Lagermontagefläche
- 48: Erhebung
- 50: Bohrung
- 52, 54: Montagefläche
- 56: Plattenabschnitt
- 58, 58': Zapfen
- 59: Montagebohrung
- 60: Stützfläche
- 62: laterale Nut
- 63: mediale Aussparung

## Patentansprüche

1. Humeruskomponente einer Schultergelenkprothese, umfassend einen Lagerkörper (38), einen Schaft (32) und einen Übergangsbereich (34), der zwischen dem Lagerkörper (38) und dem Schaft (32) angeordnet ist, sowie eine metaphysäre Basisplatte (36), die zwischen dem Übergangsbereich (34) und dem Lagerkörper (38) angeordnet ist, wobei eine Oberfläche des Übergangabereichs (34) großflächig eine Struktur in Form von diskreten Erhebungen (48) zum Ansatz von Knochenfragmenten und/oder Muskelansätzen und/oder Sehnenansätzen aufweiset, und wobei die diskreten Erhebungen (48) auf der Oberfläche des Übergangsbereiches (34) sowohl im Wesentlichen in proximal-distaler Richtung als auch im Wesentlichen in Umfangsrichtung diskret verteilt angeordnet sind, **dadurch gekennzeichnet, dass** die Oberfläche der metaphysären Basisplatte zumindest teilweise ebenfalls eine Struktur in Form von Erhebungen aufweist, wobei die Erhebungen die Form einzelner spitz zulaufender Elemente aufweisen.

2. Humeruskomponente nach Anspruch 1,
**dadurch gekennzeichnet, dass** eine anteriore und eine posteriore Oberfläche des Übergangsbereichs (34) sowie eine laterale Oberfläche des Übergangsbereichs (34) im Wesentlichen vollständig eine Struktur in Form von Erhebungen (48) aufweisen.

3. Humeruskomponente nach Anspruch 1 oder 2,
**dadurch gekennzeichnet, dass** die proximal-distale Erstreckung des Übergangsbereichs (34) wenigstens 15%, insbesondere wenigstens 20% und weiter insbesondere wenigstens 25%, der proximal-distalen Gesamtlänge der Humeruskomponente umfasst.

4. Humeruskomponente nach einem der vorstehenden Ansprüche, **dadurch gekennzeichnet, dass** die Struktur auf einem Umfangsbereich (34) eines Querschnitts senkrecht zur proximal-distalen Erstreckung der Humeruskomponente von wenigstens 240°, insbesondere wenigstens 270°, angeordnet ist.

5. Humeruskomponente nach einem der vorstehenden Ansprüche, **dadurch gekennzeichnet, dass** die Erhebungen (48) im Wesentlichen regelmäßig auf der Oberfläche des Übergangsbereichs (34) verteilt angeordnet sind.

6. Humeruskomponente nach einem der vorstehenden Ansprüche, **dadurch gekennzeichnet, dass** die Erhebungen (48) im Wesentlichen die Form von Pyramiden und/ oder Kegeln und/oder Stümpfen von Pyramiden und/oder Kegeln aufweisen.

7. Humeruskomponente nach einem der vorstehenden Ansprüche, **dadurch gekennzeichnet, dass** die Höhe der Erhebungen (48) weniger als 2.5 mm, insbesondere weniger als 2 mm, beträgt.

8. Humeruskomponente nach einem der vorstehenden Ansprüche, **dadurch gekennzeichnet, dass** die Höhe der Erhebungen (48) mehr als 0.5 mm, insbesondere mehr als 1 mm und weiter insbesondere mehr als 1.5 mm, beträgt.

9. Humeruskomponente nach einem der vorstehenden Ansprüche,
**dadurch gekennzeichnet, dass** die metaphysäre Basisplatte (36) eine Stützfläche (60) aufweist, die eine zwischen dem Lagerkörper (38) und dem Schaft (32) vorhandene Lücke zumindest teilweise derart ausfüllt, dass das Zusammenwachsen von Knochenfragmenten begünstigt wird, wobei die Stützfläche (60) zumindest teilweise ebenfalls eine Struktur in Form von Erhebungen (48) aufweist.

10. Humeruskomponente nach einem der vorstehenden Ansprüche,
**dadurch gekennzeichnet, dass** die Stützfläche (60) der metaphysären Basisplatte (36) zumindest eine Bohrung (50) aufweist.

11. Humeruskomponente nach einem der vorstehenden Ansprüche,
**dadurch gekennzeichnet, dass** die Stützfläche (60) der metaphysären Basisplatte (36) zumindest eine laterale Nut (62) aufweist.

12. Humeruskomponente nach Anspruch 11,
**dadurch gekennzeichnet, dass** die Bohrung (50) in unmittelbarer Nähe zu der lateralen Nut (62) angeordnet ist.

13. Humeruskomponente nach einem der vorstehenden Ansprüche,
**dadurch gekennzeichnet, dass** der Lagerkörper (38) und die metaphysäre Basisplatte (36) und/oder die metaphysäre Basisplatte (36) und der Übergangsbereich (34) trennbar miteinander verbunden sind.

14. Humeruskomponente nach einem der vorstehenden Ansprüche,
**dadurch gekennzeichnet, dass** der Übergangsbereich (34) frei von abstehenden Fixierungslaschen oder -ösen ausgebildet ist.

15. Schultergelenkprothese, umfassend eine Humeruskomponente, die gemäß einem der vorstehenden Ansprüche ausgebildet ist.

## Claims

1. A humeral component of a shoulder joint prosthesis, including a bearing body (38), a stem (32) and a transitional area (34) which is arranged between the bearing body (38) and the stem (32) as well as a metaphysial base plate (36) which is arranged between the transitional area (34) and the bearing body (38), wherein a surface of the transitional area (34) has a large- area structure in the form of discrete elevated portions (48) for the attachment of bone fragments and/or of muscle attachments and/or of tendon attachments; and wherein the discrete elevated portions (48) are arranged discretely distributed over the surface of the transitional area (34), both substantially in the proximal-distal direction and substantially in the peripheral direction, **characterised in that** the surface of the metaphysial base plate likewise has a structure in the form of elevated portions at least in part, with the elevated portions having the shape of individual acutely converging elements.

2. A humeral component in accordance with claim 1, **characterised in that** an anterior surface and a posterior surface of the transitional area (34) as well as a lateral surface of the transitional area (34) substantially completely have a structure in the form of elevated portions (48).

3. A humeral component in accordance with claim 1 or claim 2, **characterised in that** the proximal-distal extent of the transitional area (34) includes at least 15%, in particular at least 20%, and further in particular at least 25%, of the total proximal-distal length of the humeral component.

4. A humeral component in accordance with any one of the previous claims, **characterised in that** the structure is arranged over a peripheral area (34) of a cross-section perpendicular to the proximal-distal extent of the humeral component of at least 240°, in particular at least 270°.

5. A humeral component in accordance with any one of the previous claims, **characterised in that** the elevated portions (48) are arranged distributed substantially regularly over the surface of the transitional area (34).

6. A humeral component in accordance with any one of the previous claims, **characterised in that** the elevated portions (48) substantially have the form of pyramids and/or of cones and/or of truncated pyramids and/or of truncated cones.

7. A humeral component in accordance with any one of the previous claims, **characterised in that** the height of the elevated portions (48) amounts to less than 2.5 mm, in particular to less than 2 mm.

8. A humeral component in accordance with any one of the previous claims, **characterised in that** the height of the elevated portions (48) amounts to more than 0.5 mm, in particular to more than 1 mm and further in particular to more than 1.5 mm.

9. A humeral component in accordance with any one of the previous claims, **characterised in that** the metaphysial base plate (36) has a support surface (60) which fills a gap between the bearing body (38) and the stem (32) at least in part such that the fusion of bone fragments is promoted, with the support surface (60) likewise having a structure in the form of elevated portions (48) at least in part.

10. A humeral component in accordance with any one of the previous claims, **characterised in that** the support surface (60) of the metaphysial base plate (36) has at least one bore (50).

11. A humeral component in accordance with any one of the previous claims, **characterised in that** the support surface (60) of the metaphysial base plate (36) has at least one lateral groove (62).

12. A humeral component in accordance with claim 11, **characterised in that** the bore (50) is arranged in direct proximity to the lateral groove (62).

13. A humeral component in accordance with any one of the previous claims, **characterised in that** the bearing body (38) and the metaphysial base plate (36) and/or the metaphysial base plate (36) and the transitional area (34) are separably connected to one another.

14. A humeral component in accordance with any one of the previous claims, **characterised in that** the transitional area (34) is free from projecting fixing lugs or fixing eyelets.

15. A shoulder joint prosthesis, including a humeral component which is made in accordance with any one of the preceding claims.

## Revendications

1. Composant huméral d'une prothèse d'articulation d'épaule, comprenant un corps de montage (38), une tige (32) et une zone de transition (34) qui est agencée entre le corps de montage (38) et la tige (32), ainsi qu'une plaque de base métaphysaire (36) qui est agencée entre la zone de transition (34) et le corps de montage (38), une surface de la zone de transition (34) présentant sur une grande étendue une structure sous forme de reliefs (48) discrets pour l'accrochage de fragments osseux et/ou de muscles et/ou de tendons, et les reliefs discrets (48) étant agencés en répartition discrète sur la surface de la zone de transition (34) tant sensiblement en direction proximale-distale que sensiblement en direction périphérique, **caractérisé en ce que** la surface de la plaque de base métaphysaire présente au moins partiellement également une structure sous forme de reliefs, les reliefs présentant la forme d'éléments individuels se terminant en pointe.

2. Composant huméral selon la revendication 1,
**caractérisé en ce qu'**une surface antérieure et une surface postérieure de la zone de transition (34) ainsi qu'une surface latérale de la zone de transition (34) présentent sensiblement complètement une structure sous forme de reliefs (48).

3. Composant huméral selon la revendication 1 ou 2,
**caractérisé en ce que** l'extension proximale-distale de la zone de transition (34) comprend au moins 15 %, en particulier au moins 20 % et plus particulièrement au moins 25 % de la longueur totale proximale-distale du composant huméral.

4. Composant huméral selon l'une quelconque des revendications précédentes,
**caractérisé en ce que** la structure est agencée sur une zone périphérique (34) d'au moins 240°, en particulier d'au moins 270°, d'une section transversale perpendiculairement à l'extension proximale-distale du composant huméral.

5. Composant huméral selon l'une quelconque des revendications précédentes,
**caractérisé en ce que** les reliefs (48) sont agencés de manière sensiblement régulière sur la surface de la zone de transition (34).

6. Composant huméral selon l'une quelconque des revendications précédentes,
**caractérisé en ce que** les reliefs (48) présentent sensiblement la forme de pyramides et/ou de cônes et/ou de troncs de pyramide et/ou de troncs de cône.

7. Composant huméral selon l'une quelconque des revendications précédentes,
**caractérisé en ce que** la hauteur des reliefs (48) est inférieure à 2,5 mm, en particulier inférieure à 2 mm.

8. Composant huméral selon l'une quelconque des revendications précédentes,
**caractérisé en ce que** la hauteur des reliefs (48) est supérieure à 0,5 mm, en particulier supérieure à 1 mm et plus particulièrement supérieure à 1,5 mm.

9. Composant huméral selon l'une quelconque des revendications précédentes,
**caractérisé en ce que** la plaque de base (36) métaphysaire présente une surface d'appui (60) qui remplit au moins partiellement une lacune se trouvant entre le corps de montage (38) et la tige (32), de telle sorte que le soudage de fragments osseux est favorisé, la surface d'appui (60) présentant au moins partiellement également une structure sous forme de reliefs (48).

10. Composant huméral selon l'une quelconque des revendications précédentes,
**caractérisé en ce que** la surface d'appui (60) de la plaque de base (36) métaphysaire présente au moins un perçage (50).

11. Composant huméral selon l'une quelconque des revendications précédentes,
**caractérisé en ce que** la surface d'appui (60) de la plaque de base (36) métaphysaire présente au moins une gorge (62) latérale.

12. Composant huméral selon la revendication 11,
**caractérisé en ce que** le perçage (50) est agencé à proximité directe de la gorge (62) latérale.

13. Composant huméral selon l'une quelconque des revendications précédentes,
**caractérisé en ce que** le corps de montage (38) et la plaque de base (36) métaphysaire et/ou la plaque de base (36) métaphysaire et la zone de transition (34) sont reliés mutuellement de manière séparable.

14. Composant huméral selon l'une quelconque des revendications précédentes,
**caractérisé en ce que** la zone de transition (34) est réalisée sans pattes ou oeillets de fixation faisant saillie.

15. Prothèse d'articulation d'épaule, comprenant un composant huméral qui est réalisé selon l'une quelconque des revendications précédentes.
